# EUROPEAN PATENT SPECIFICATION

(11) **EP 1 272 663 B1**
(45) Date of publication and mention of the grant of the patent: **30.12.2009**
(21) Application number: 01913809.8
(22) Date of filing: 09.02.2001
(51) Int. Cl.: C12Q 1/68, C07K 14/80, C07K 16/18, C12N 5/10, C12N 15/63, C12N 9/02, C12N 15/53, A01K 67/027, A61K 48/00, A61K 31/00, A61K 38/46, G01N 33/53

(54) **POLYMORPHISMS IN THE HUMAN CYP2B6 GENE AND THEIR USE IN DIAGNOSTIC AND THERAPEUTIC APPLICATIONS**
POLYMORPHISMEN DES HUMANEN CYP2B6 GENS UND DEREN VERWENDUNG IN DIAGNOSTISCHEN UND THERAPEUTISCHEN ANWENDUNGEN
POLYMORPHISMES DANS LE GENE HUMAIN CYP2B6 ET LEUR UTILISATION DANS DES APPLICATIONS DIAGNOSTIQUES ET THERAPEUTIQUES

(30) Priority: 09.02.2000 EP 00102701
(43) Date of publication of application: 08.01.2003
(73) Proprietor: Epidauros Biotechnologie AG, 82347 Bernried (DE)
(72) Inventor: ZANGER, Ulrich, M., 70825 Korntal (DE); LANG, Thomas, 73235 Weilheim an der Teck (DE)
(74) Representative: Dörries, Hans Ulrich
(86) International application number: PCT/EP2001/001456
(87) International publication number: WO 2001/059152

(56) References cited:
- EP-A- 0 644 267
- WO-A-92/14817
- WO-A-99/05299
- WO-A-99/45126
- US-A- 5 688 773
- SHIGERU YAMANO ET AL: "CDNA CLONING AND SEQUENCE AND CDNA-DIRECTED EXPRESSION OF HUMAN P450 IIB1: IDENTIFICATION OF A NORMAL AND TWO VARIANT CDNAS DERIVEDFROM THE CYP2B LOCUS ON CHROMOSOME 19 AND DIFFERENTIAL EXPRESSION OF THE IIB MRNAS IN HUMAN LIVER" BIOCHEMISTRY, AMERICAN CHEMICAL SOCIETY. EASTON, PA, US, vol. 28, no. 18, 5 September 1989 (1989-09-05), pages 7340-7348, XP000569732 ISSN: 0006-2960 cited in the application
- MILES J ET AL: "Alternative splicing in the human cytochrome P450IIB6 gene generates a high level of aberrant messages" NUCLEIC ACIDS RESEARCH, vol. 17 , no. 20, 1989, pages 8241-55, XP002196320
- MILES J ET AL: "A novel human cytochrome P450 gene (P450IIB): chromosomal location and evidence for alternative splicing" NUCLEIC ACIDS RESEARCH, vol. 16, no. 3, 1988, pages 5783-95, XP002196321
- EKINS S ET AL: "THE ROLE OF CYP2B6 IN HUMAN XENOBIOTIC METABOLISM" DRUG METABOLISM REVIEWS, MARCEL DEKKER, NEW YORK, NY, US, vol. 31, no. 3, August 1999 (1999-08), pages 719-754, XP001037410 ISSN: 0360-2532
- LANG T ET AL: "Extensive genetic polymorphism in the human CYP2B6 gene with impact on expression and function in human liver" PHARMACOGENETICS, vol. 11, no. 5, July 2001 (2001-07), pages 399-415, XP002196863

## Description

### Field of the invention

The present invention relates generally to means and methods of diagnosing the phenotypic spectrum as well as the overlapping clinical characteristics with several forms of inherited abnormal expression and/or function of the cytochrome P-450 (CYP)2B6 gene. In particular, the present invention relates to a method of diagnosing drug hypersensitivity related to the presence of a molecular variant of the CYP2B6 gene comprising determining in a sample from a subject the presence of a polynucleotide selected from the group consisting of:
(a) a polynucleotide encoding a CYP2B6 polypeptide or fragment thereof, wherein said polypeptide comprises an amino acid substitution of Gln172 to His in exon 4 and/or Arg487 to Cys in exon 9 of the CYP2B6 polypeptide having accession No: M29874; and
(b) a polynucleotide capable of hydridizing to the CYP2B6 gene, wherein said polynucleotide is having at a position corresponding to position 516 or 1459 of the CYP2B6 gene having accession No: M29874, wherein nucleotide A at position 7 has been numbered +1 a T;
wherein the presence of said polynucleotide or fragment thereof is considered indicative of said drug hypersensitivity in said method.

In another aspect, the present inventon relates to a use of an oligo- or polynucleotide for diagnosing drug hypersensitivity related to the presence of a molecular variant of the CYP2B6 gene comprising determining in a sample from a subject the presence of a polynucleotide selected from the group consisting of:
(a) a polynucleotide encoding a CYP2B6 polypeptide or fragment thereof, wherein said polypeptide comprises an amino acid substitution of Gln172 to His in exon 4 and/or Arg487 to Cys in exon 9 of the CYP2B6 polypeptide having accession No: M29874; and
(b) a polynucleotide capable of hybridizing to the CYP2B6 gene, wherein said polynucleotide is having at a position corresponding to position 516 or 1459 of the CYP2B6 gene having accession No: M29874, wherein nucleotide A at position 7 has been numbered +1 a T.

In a further aspect, the present invention relates to an in vitro method for the development of a pharmacodynamic profile of drugs that are substrates of the CYP2B6 gene product for a patient or for improving the therapy of disorders with drugs that are targets of the CYP2B6 gene product comprising using the CYP2B6 gene single nucleotide polymorphism at position 516, or 1459 as defined in claim 1.

### Background of the invention

Members of the cytochrome P-450 (CYP) family of hemoproteins metabolise a wide variety of endogenous substrates such as steroid hormones, and of xenobiotics including carcinogens, toxins and drugs; see, e.g., Daly, Toxicol. Lett. 102-103 (1998), 143-147; Touw, Drug Metabol. Drug Interact. 14 (1997), 55-82. CYP2B6 is involved in the metabolic activation and inactivation of a number of clinically important drugs such as the frequently used antineoplastic agents cyclophosphamide and ifosfamide, the antioestrogen tamoxifen, the newer platelet aggregation inhibitor Clopidogrel, but also envirenmental toxicants and recreational drugs such as nicotine and ecstasy. The CYP2B6 gene has been mapped to chromosome 19 between 19q12 and 19q13.2 (Miles et al., 1989; Carrano et al., 1989; Hoffman et al., 1995). It spans a region of approximately 28 kb and like other members of the CYP2 family it contains 9 exons which are separated by 8 introns. - The gene encodes a microsomal protein with 491 amino acids. The CYP2B6 gene is located within a 350 kb gene cluster together with a number of other genes and pseudogenes of the CYP2A, 2B, and 2F subfamilies (Hoffman et al., 1995). The CYP2B subfamily consists of the functional CYP2B6 gene, the nonfunctional CYP2B7 gene and a CYP2B7-like pseudogene (Yamano 1989; Hoffman 1995). CYP2B6 mRNA and protein are primarily expressed in human liver where it contributes an estimated 1 to 5 % to the total liver content of cytochrome P450 (Gervot et al., 1999). Extrahepatic expression occurs at lower levels in kidney, intestine and lung (Gervot et al., 1999; Gonzalez et al., 1992). Initially, it was thought that CYP2B6 is expressed in only about 25% of all human livers (Mimura et al., 1993). However, with new antibodies of higher sensitivity CYP2B6 was detectable in all liver specimens with an interindividual variability of expression of up to 100-fold (Code et al., 1997; Gervot et al., 1999). The reasons for this high variability are unknown but most likely two mechanisms are involved:
(a) enzyme induction: CYP2B6 is the human homologe to the rat P450s CYP2B1 /CYP2B2 which are known to be inducible by phenobarbital (Sueyoshi et al., 1999);
(b) genetic polymorphism: some limited indications for existing genetic polymorphisms, including a polymorphic Bam HI and Bgl II site observed as RFLP were described in one of the original publications (Miles et al., 1988; Yamano et al. 1989 ).

It is clear that naturally occurring mutations, if they exist can have effects on drug metabolization and efficacy of therapies with drugs, for example in cancer treatment. It is unknown, however, how many of such variations exist, and with what frequency and at what positions in the human CYP2B6 gene.
Accordingly, means and methods for diagnosing and treating a variety of forms of individual drug intolerability and inefficacy of drug therapy which result from CYP2B6 gene polymorphisms that interfere e.g., with chemotherapeutic treatment of diseases, was hitherto not available but are nevertheless highly desirable.

Thus, the technical problem of the present invention is to comply with the needs described above. - The solution to this technical problem is achieved by providing the embodiments characterized in the claims.

### Summary of the Invention

The present invention is based on the finding of novel, so far unknown variations in the nucleotide sequence of the CYP2B6 gene and the population distribution of these alleles. Based upon the knowledge of these novel sequences diagnostic tests and reagents for such tests were designed for the specific detection and genotyping of CYP2B6 alleles in humans, including homozygous as well as heterozygous, frequent as well as rare alleles of the CYP2B6 genes. The determination of the CYP2B6 gene allele status of humans with such tests is useful for the optimization of therapies with the numerous substrates of CYP2B6. It may also be useful in the determination of the individual predisposition to several common cancers caused by environmental carcinogens.
In a first embodiment, the invention provides a method of diagnosing drug hypersensitivity related to the presence of a molecular variant of the CYP2B6 gene comprising determining in a sample from a subject the presence of a polynucleotide selected from the group consisting of:
(a) a polynucleotide encoding a CYP2B6 polypeptide or fragment thereof, wherein said polypeptide comprises an amino acid substitution of Gln172 to His in exon 4 and/or Arg487 to Cys in exon 9 of the CYP2B6 polypeptide having accession No: M29874; and
(b) a polynucleotide capable of hydridizing to the CYP2B6 gene, wherein said polynucleotide is having at a position corresponding to position 516 or 1459 of the CYP2B6 gene having accession No: M29874, wherein nucleotide A at position 7 has been numbered +1 a T;
wherein the presence of said polynucleotide or fragment thereof is considered indicative of said drug hypersensitivity in said method.

In another aspect, the present inventon relates to a use of an oligo- or polynucleotide for diagnosing drug hypersensitivity related to the presence of a molecular variant of the CYP2Bti gene comprising determining in a sample from a subject the presence of a polynucleotide selected from the group consisting of:
(a) a polynucleotide encoding a CYP2B6 polypeptide or fragment thereof, wherein said polypeptide comprises an amino acid substitution of Gln172 to His in exon 4 and/or Arg487 to Cys in exon 9 of the CYP2B6 polypeptide having accession No: M29874; and
(b) a polynucleotide capable of hybridizing to the CYP2B6 gene, wherein said polynucleotide is having at a position corresponding to position 516 or 1459 of the CYP2B6 gene having accession No: M29874, wherein nucleotide A at position 7 has been numbered +1 a T; and

In a further aspect, the present invention relates to an in vitro method for the development of a pharmacodynamic profile of drugs that are substrates of the CYP2B6 gene product for a patient or for improving the therapy of disorders with drugs that are targets of the CYP2B6 gene product comprising using the CYP2B6 gene single nucleotide polymorphism at position 516, or 1459 as defined in claim 1.

The diagnostic methods and uses of the invention are useful for the diagnosis and treatment of cancer and other diseases the therapy of which is dependent on drug treatment and tolerance. The novel variant forms of CYP2B6 genes according to the invention provide the potential for the development of a pharmacodynamic profile of drugs for a given patient.

### Description of the invention

The finding and characterization of variations in the CYP2B6 gene, and diagnostic tests for the discrimination of different CYP2B6 alleles in human individuals provide a very potent tool for improving the therapy of diseases with drugs that are targets of the CYP2B6 gene product, and whose metabolization is therefore dependent on CYP2B6. The diagnosis of the Individual allelic CYP2B6 status permits a more focused therapy, e.g., by opening the possibility to apply individual dose regimens of drugs. It may also be useful as prognostic tool for therapy outcome. Furthermore, diagnostic tests to genotype CYP2B6, and novel CYP2B6 variants, will not only improve therapy with established drugs and help to correlate genotypes with drug activity or side effects. These tests and sequences also provide reagents for the development of novel inhibitors that specifically modulate the activity of the individual types of CYP2B6. Expression in yeast, for example, of three allelic cDNAs encoding human liver CYP3A4 and methods for testing the binding properties and catalytic activities of their gene products have been described in Peyronneau, Eur. J. Biochem. 218 (1993).
Thus, the present invention provides a novel way to exploit molecular biology and pharmacological research for drug therapy while bypassing their potential detrimental effects which are due to expression of variant CYP2B6 genes.

Accordingly, the invention relates to a method of diagnosing drug hypersensitivity related to the presence of a molecular variant of the CYP2B6 gene comprising determining in a sample from a subject the presence of a polynucleotide selected from the group consisting of:
(a) a polynucleotide encoding a CYP2B6 polypeptide or fragment thereof, wherein said polypeptide comprises an amino acid substitution of Gln172 to His in exon 4 and/or Arg487 to Cys in exon 9 of the CYP2B6 polypeptide having accession No: M29874; and
(b) a polynucleotide capable of hydridizing to the CYP2B6 gene, wherein said polynucleotide is having at a position corresponding to position 516 or 1459 of the CYP2B6 gene having accession No: M29874, wherein nucleotide A at position 7 has been numbered +1 a T;
wherein the presence of said polynucleotide or fragment thereof is considered indicative of said drug hypersensitivity in said method.

In another aspect, the present inventon relates to a use of an oligo- or polynucleotide for diagnosing drug hypersensitivity related to the presence of a molecular variant of the CYP2B6 gene comprising determining in a sample from a subject the presence of a polynucleotide selected from the group consisting of:
(a) a polynucleotide encoding a CYP2B6 polypeptide or fragment thereof, wherein said polypeptide comprises an amino acid substitution of Gln172 to His in exon 4 and/or Arg487 to Cys in exon 9 of the CYP2B6 polypeptide having accession No: M29874; and
(b) a polynucleotide capable of hybridizing to the CYP2B6 gene, wherein said polynucleotide is having at a position corresponding to position 516 or 1459 of the CYP2B6 gene having accession No: M29874, wherein nucleotide A at position 7 has been numbered +1 a T; and

In a further aspect, the present invention relates to an in vitro method for the development of a pharmacodynamic profile of drugs that are substrates of the CYP2B6 gene product for a patient or for improving the therapy of disorders with drugs that are targets of the CYP2B6 gene product comprising using the CYP2B6 gene single nucleotide polymorphism at position 516, or 1459 as defined in claim 1.

The variant polynucleotide and polypeptides applied in the methods and uses of the invention also comprise fragments of said polynucleotides or polypeptides of the invention. The polynucleotides and polypeptides as well as the aforementioned fragments thereof applied in the methods and uses of the invention are characterized as being associated with altered biochemical properties of the CYP2B6 protein and/or altered expression of the variant CYP2B6 gene compared to the corresponding wild type gene. Said altered biochemical properties are considered within the gist of the present invention to lead to altered biochemical properties of the CYP2B6 protein such as protein activity and therefore are considered to lead to interindividual differences in drug metabolism.

In the context of the present invention the term "molecular variant" CYP2B6 gene or protein as used herein means that said CYP2B6 gene or protein differs from the wild type CYP2B6 gene or protein by way of nucleotide substitutlon(s), addition(s) and/or deletion(s) (partly genomic and cDNA sequences of the CYP2B6 gene are described in, for example Miles et al., 1988; Miles et al.,1989; Miles et al., 1990; Yamano et al., 1989; Sueyoshi et al.,1999; accession numbers:J02864; X13494; M29874; X06399; X16864; X06400; Af081569). Preferably, said nucleotide substitution(s) result(s) in a corresponding change in the amino acid sequence of the CYP2B6 protein.

The term "hybridizing" as used herein refers to polynucleotides applied in the methods and uses of the invention which are capable of hybridizing to the polynucleotides of the invention or parts thereof which are associated with altered biochemical properties of the CYP2B6 protein and/or altered expression of the variant CYP2B6 gene compared to the corresponding wild type gene. Thus, said hybridizing polynucleotides are also associated with said altered biochemical properties of the CYP2B6 protein and/or said altered expression of the variant CYP2B6 gene compared to the corresponding wild type gene. Therefore, said polynucleotides may be useful as probes In Northern or Southern Blot analysis of RNA or DNA preparations, respectively, or can be used as oligonucleotide primers In PCR analysis dependent on their respective size. - Also comprised are hybridizing polynucleotides which are useful for analysing DNA-Protein interactions via, e.g., electrophoretic mobility shift analysis (EMSA). Preferably, said hybridizing polynucleotides comprise at least 10, more preferably at least 15 nucleotides in length while a hybridizing polynucleotide of the present invention to be used as a probe preferably comprises at least 100, more preferably at least 200, or most preferably at least 500 nucleotides in length.
It is well known in the art how to perform hybridization experiments with nucleic acid molecules, i.e. the person skilled in the art knows what hybridization conditions s/he has to use in accordance with the present invention. Such hybridization conditions are referred to in standard text books such as Molecular Cloning A Laboratory Manual, Cold Spring Harbor Laboratory (1989) N.Y. Preferred in accordance with methods and uses of the present invention are polynucleotides which are capable of hybridizing to the polynucleotides of the invention or parts thereof which are associated with altered biochemical properties of the CYP2B6 protein and/or altered expression of the variant CYP2B6 gene compared to the corresponding wild type gene under stringent hybridization conditions, i.e. which do not cross hybridize to unrelated polynucleotides such as polynucleotides that may not alter biochemical properties of the CYP2B6 protein and/or alter expression of the variant CYP2B6 gene compared to the corresponding wild type gene.

The term "corresponding" as used herein means that a position is not only determined by the number of the preceding nucleotides and amino acids, respectively. The position of a given nucleotide or amino acid In accordance with the present invention which may be deleted, substituted or comprise one or more additional nucleotide(s) may vary due to deletions or additional nucleotides or amino acids elsewhere in the promotor and/or gene. Thus, under a "corresponding position" in accordance with the present invention it is to be understood that nucleotides or amino acids may differ in the indicated number but may still have similar neighboring nucleotide or amino acids. Said nucleotide or amino acids which may be exchanged, deleted or comprise additional nucleotides or amino acids are also comprised by the term "corresponding position". Said nucleotides or amino acids may for instance together with their neighbors form sequences which may be involved in the regulation of gene expression, stability of the corresponding RNA or RNA editing, as well as functional domains or motifs of the protein of the invention.

In accordance with the present invention, the mode and population distribution of novel so far unidentified genetic variations in the CYP2B6 gene have been analyzed by sequence analysis of relevant regions of the human CYP2B6 gene from many different individuals. Except for some limited initial observations which indicated the occurrence of genetic polymorphisms of the CYP2B6 gene (Miles et al., 1989; Yamano et al., 1989), no systematic analysis has been carried out. This study was undertaken in accordance with the present invention as the first systematic approach to detect genetic polymorphisms in the human CYP2B6 gene and, surprisingly, revealed that mutations In the CYP2B6 gene exist, in particular in the coding regions of the gene that can be expected to cosegregnate and optionally lead to altered biochemical properties of the CYP2B6 protein such as protein stability, activity, or substrate specificity and will lead to interindividual differences in drug metabolism. It is a well known fact that genomic DNA of individuals, which harbor the individual genetic makeup of all genes, including CYP2B6 can easily be purified from individual blood samples. These individual DNA samples are then used for the analysis of the sequence composition of the CYP2B6 gene alleles that are present in the individual which provided the blood sample. The sequence analysis was carried out by PCR amplification of relevant regions of the CYP2B6 gene, subsequent purification of the PCR products, followed by automated DNA sequencing with established methods; see the Examples.
One important parameter that had to be considered in the attempt to determine the individual CYP2B6 genotype and identify novel CYP2B6 variants by direct DNA-sequencing of PCR-products from human blood genomic DNA is the fact that each human harbors (usually, with very few abnormal exceptions) two gene copies of each autosomal gene (diploidy). Because of that, great care had to be taken in the evaluation of the sequences to be able to identify unambiguously not only homozygous sequence variations but also heterozygous variations. The details of the different steps In the identification and characterization of novel CYP2B6 gene polymorphisms (homozygous and heterozygous) are described in the examples - below.

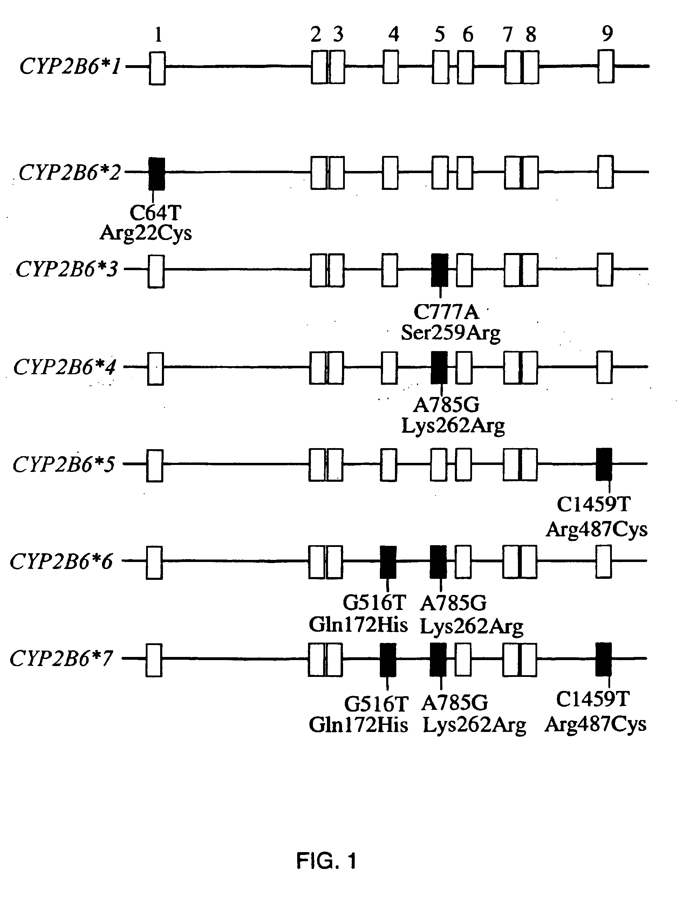

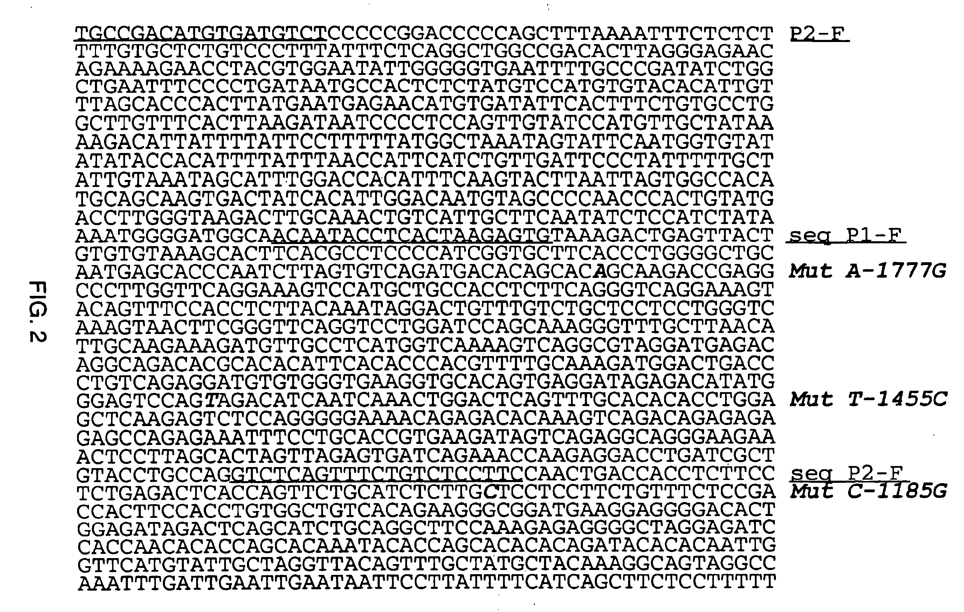

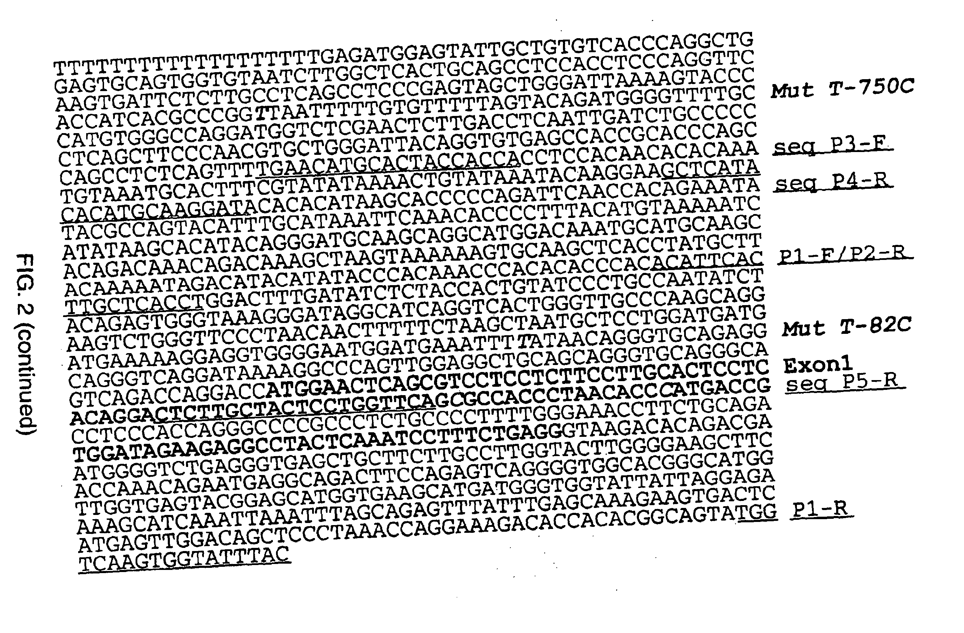

The mutations in the CYP2B6 genes detected in accordance with the present invention are illustrated in Figure 1 and 2 and in Tables 2 and 3, respectively. The methods of the mutation analysis followed standard protocols and are described in detail in the examples. In general such methods to be used in accordance with the present invention for evaluating the phenotypic spectrum as well as the overlapping clinical characteristics with other forms of drug metabolization and altered tolerance to drugs in patients with mutations in the CYP2B6 gene encompass for example haplotype analysis, single-strand conformation polymorphism analysis (SSCA), PCR and direct sequencing. On the basis of thorough clinical characterization of many patients the phenotypes can then be correlated to these mutations as well as to mutations that had been described earlier for other CYPs.

As is evident to the person skilled in the art this new molecular genetic knowledge can now be used to exactly characterize the genotype of the index patient where a given drug takes an unusual effect and of his family.

Over the past 20 years, genetic heterogeneity has been increasingly recognized as a significant source of variation in drug response. Many scientific communications (Meyer, Ann. Rev. Pharmacol. Toxicol. 37 (1997), 269-296 and West, J. Clin. Pharmacol. 37 (1997), 635-648) have clearly shown that some drugs work better or may even be highly toxic in some patients than in others and that these variations in patient's responses to drugs can be related to molecular basis. This "pharmacogenomic" concept spots correlations between responses to drugs and genetic profiles of patient's (Marshall, Nature Biotechnology, 15 (1997), 954-957; Marshall, Nature Biotechnology, 15 (1997), 1249-1252). In this context of population variability with regard to drug therapy, pharmacogenomics has been proposed as a tool useful in the identification and selection of patients which can respond to a particular drug without side effects. This identification/selection can be based upon molecular diagnosis of genetic polymorphisms by genotyping DNA from leukocytes in the blood of patient, for example, and characterization of - disease (Bertz, Clin. Pharmacokinet. 32 (1997), 210-256; Engei, J. Chromatogra. B. Biomed. Appl. 678 (1996), 93-103). For the providers of health care, such as health maintenance organizations in the US and government public health services in many European countries, this pharmacogenomics approach can represent a way of both improving health care and reducing overheads because there is a large cost to unnecessary therapies, ineffective drugs and drugs with side effects.

The mutations in the variant CYP2B6 gene found by the inventors result in amino acid deletion(s), insertion(s) and In particular in substitution(s) either alone or in combination. It is of course also possible to genetically engineer such mutations in wild type genes or other mutant forms. Methods for introducing such modifications in the DNA sequence of CYP2B6 gene are well known to the person skilled In the art; see, e.g., Sambrook, Molecular Cloning A Laboratory Manual, Cold Spring Harbor Laboratory (1989) N.Y.

For the investigation of the nature of the alterations in the amino acid sequence of the CYP2B6 proteins computer programs may be used such as RASMOL that are obtainable from the internet. Furthermore, folding simulations and computer redesign of structural motifs can be performed using other appropriate computer programs (Olszewski, Proteins 25 (1996), 286-299; Hoffman, Comput. Appl. Biosci. 11 (1995), 675-679). Computers can be used for the conformational and energetic analysis of detailed protein models (Monge, J. Mol. Biol. 247 (1995), 995-1012; Renouf, Adv. Exp. Med. Biol. 376 (1995), 37-45). These analysis can be used for the identification of the influence of a particular mutation on binding and/or metabolization of drugs.

Usually, said amino acid substitution in the amino acid sequence of the protein encoded by the polynucleotide applied in the methods and uses of the invention is due to one or more nucleotide substitution. Preferably, said nucleotide substitution results in an amino acid substitution of Gln172 to His in exon 4 and/or Arg487 to Cys in exon 9 of the CYP2B6 gene.

The polynucleotide applied in the methods and uses of the invention may further comprise at least one nucleotide and optionally amino acid deletion, addition and/or substitution in its encoded protein other than those specified hereinabove. This embodiment of the present invention allows the study of synergistic effects of the mutations in the CYP gene on the pharmacological profile of drugs in patients who bear such mutant forms of these genes or similar mutant forms that can be mimicked by the above described proteins. It is expected that the analysis of said synergistic effects provides deeper insights into drug tolerant or sensitive phenotypes of certain forms of cancer and other diseases. From said deeper insight the development of diagnostic and pharmaceutical compositions related to cancer will greatly benefit.

The nucleotide substitution in the polynucleotide applied in the methods and uses of the invention results in altered expression of the variant CYP2B6 gene compared to the corresponding wild type gene.

The polynucleotide applied in the methods and uses of the invention comprises a nucleotide sequence encoding a molecular variant of the cytochrome P450 (CYP)2B6 protein by way of at least one amino acid substitution at an amino acid position corresponding amino acid residue Gln172 in exon 4 and/or Arg487 in exon 9 of CYP2B6 gene.

The polynucleotide applied in the methods and uses of the invention may be, e.g., DNA, cDNA, genomic DNA, RNA or synthetically produced DNA or RNA or a recombinantly produced chimeric nucleic acid molecule comprising any of those polynucleotides either alone or in combination. Preferably said polynucleotide may by part of a vector, particularly plasmids, cosmids, viruses and bacteriophages used conventionally in genetic engineering that comprise a polynucleotide of the invention. Such vectors may comprise further genes such as marker genes which allow for the selection of said vector in a suitable host cell and under suitable conditions.

It is further preferred that the vector or the polynucleotide is operatively linked to expression control sequences allowing expression in prokaryotic or eukaryotic cells. Expression of said polynucleotide comprises transcription of the polynucleotide, preferably into a translatable mRNA. Regulatory elements ensuring expression in eukaryotic cells, preferably mammalian cells, are well known to those skilled in the art. They usually comprise regulatory sequences ensuring initiation of transcription and optionally poly-A signals ensuring termination of transcription and stabilization of the transcript. Additional regulatory elements may include transcriptional as well as translational enhancers. Possible regulatory elements permitting expression in prokaryotic host cells comprise, e.g., the *lac, trp* or *tac* promoter in *E. coli,* and examples for regulatory elements permitting expression in eukaryotic host cells are the *AOX1* or *GAL1* promoter in yeast or the CMV-, SV40- , RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pCDM8, pRc/CMV, pcDNA1, pcDNA3 (In-vitrogene), pSPORT1 (GIBCO BRL). Preferably, said vector is an expression vector and/or a gene transfer or targeting vector. Expression vectors - derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994). Alternatively, the polynucleotides and vectors of the invention can be reconstituted into liposomes for delivery to target cells.

Also described are host cells transformed with a polynucleotide or vector as described herein. Said host cell may be a prokaryotic or eukaryotic cell; see supra. The polynucleotide or vector which is present in the host cell may either be integrated into the genome of the host cell or it may be maintained extrachromosomally. In this respect, it is also to be understood that the recombinant DNA molecule of the invention can be used for "gene targeting" and/or "gene replacement", for restoring a mutant gene or for creating a mutant gene via homologous recombination, see for example Mouellic, Proc. Natl. Acad. Scl. USA, 87 (1990), 4712-4716; Joyner, Gene Targeting, A Practical Approach, Oxford University Press.
The host cell can be any prokaryotic or eukaryotic cell, such as a bacterial, insect, fungal, plant, animal or human cell. Preferred fungal cells are, for example, those of the genus Saccharomyces, in particular those of the species S. cerevisiae. The term "prokaryotic" is meant to include all bacteria which can be transformed or transfected with a polynucleotide for the expression of a variant CYP2B6 protein or fragment thereof. Prokaryotic hosts may include gram negative as well as gram positive bacteria such as, for example, *E. coli, S. typhimurium, Serratia marcescens* and *Bacillus subtilis*. A polynucleotide coding for a mutant form of CYP2B6 variant proteins can be used to transform or transfect the host using any of the techniques commonly known to those of ordinary skill in the art. Methods for preparing fused, operably linked genes and expressing them in bacteria or animal cells are well-known in the art (Sambrook, supra). The genetic constructs and methods described therein can be utilized for expression of variant CYP2B6 proteins in, e.g., prokaryotic hosts. In general, expression vectors containing promoter sequences which facilitate the efficient transcription of the inserted polynucleotide are used in connection with the host. The expression vector typically contains an origin of replication, a promoter, and a terminator, as well as specific genes which are capable of providing phenotypic selection of the transformed cells. The transformed prokaryotic hosts can be grown in fermentors and cultured according to techniques known in the art to achieve optimal cell growth. The proteins of the invention can then be isolated from the grown medium, cellular lysates, or cellular membrane fractions. The isolation and purification of the microbially or otherwise expressed polypeptides of the invention may be by any conventional means such as, for example, preparative chromatographic separations and immunological separations such as those involving the use of monoclonal or polyclonal antibodies.

Thus, also described herein is a method for the production of variant CYP2B6 proteins and fragments thereof comprising culturing a host cell as defined above under conditions allowing the expression of the protein and recovering the produced protein or fragment from the c :ure.

Further described herein is a method for producing cells capable of expressing a variant CYP2B6 gene comprising genetically engineering cells with the polynucleotide or with the vector as described herein. The cells obtainable by the method of the invention can be used, for example, to test drugs according to the methods described in Sambrook, Fritsch, Maniatis (1989). Molecular cloning: a laboratory manual. Cold Spring Harbour Laboratory press, Cold Spring Harbour; Peyronneau, Eur J Biochem 218 (1993), 355-61; Yamazaki, Carcinogenesis 16 (1995), 2167-2170. Furthermore, the cells can be used to study known drugs and unknown derivatives thereof for their ability to complement loss of drug efficacy caused by mutations in the CYP2B6 gene. For these purposes the host cells preferably lack a wild type allele, preferably both alleles of the CYP2B6 gene and/or have at least one mutated from thereof. Alternatively, strong overexpression of a mutated allele over the normal allele and comparison with a recombinant cell line overexpressing the normal allele at a similar level may be used as a screening and analysis system. The cells obtainable by the above-described method may also be used for screening methods.

Furthermore, herein described are variant CYP2B6 protein and fragments thereof encoded by a polynucleotide described herein or obtainable by the above-described method or from cells produced by the method described above.

Furthermore, in the methods and uses of the invention nucleic acid molecules which represent or comprise the complementary strand of any of the above described polynucleotides or a part thereof, thus comprising at least one nucleotide difference compared to the corresponding wild type CYP2B6 gene nucleotide sequences specified by the above described nucleotide substitutions can be applied.
Such a molecule may either be a deoxyribonucleic acid or a ribonucleic acid. Such molecules comprise, for example, antisense RNA.

With the variant CYP2B6 polynucleotides and proteins and vectors described herein, it is now possible to study *in vivo* and *in vitro* the efficiency of drugs in relation to particular mutations in the CYP2B6 gene of a patient and the affected phenotype. Furthermore, the variant CYP2B6 proteins described herein can be used to determine the pharmacological profile of drugs and for the identification and preparation of further drugs which may be more effective for the treatment of, e.g., cancer, in particular for the amelioration of certain phenotypes caused by the respective mutations such as those described above.

The presence or expression of variant CYP2B6 genes can be monitored by using a primer pair that specifically hybridizes to either of the corresponding nucleic acid sequences and by carrying out a PCR reaction according to standard procedures. Specific hybridization of the above mentioned probes or primers preferably occurs at stringent hybridization conditions. The term "stringent hybridization conditions" is well known in the art; see, for example, Sambrook et al., "Molecular Cloning, A Laboratory Manual" second ed., CSH Press, Cold Spring Harbor, 1989; "Nucleic Acid Hybridisation, A Practical Approach", Hames and Higgins eds., IRL Press, Oxford, 1985. Furthermore, the mRNA, cRNA, cDNA or genomic DNA obtained from the subject may be sequenced to identify mutations which may be characteristic fingerprints of mutations in the CYP2B6 gene. The present invention further comprises methods wherein such a fingerprint may be generated by RFLPs of DNA or RNA obtained from the subject, optionally the DNA or RNA may be amplified prior to analysis, the methods of which are well known in the art. RNA fingerprints may be performed by, for example, digesting an RNA sample obtained from the subject with a suitable RNA-Enzyme, for example RNase T₁, RNase T₂ or the like or a ribozyme and, for example, electrophoretically separating and detecting the RNA fragments as described above.
Further modifications of the above-mentioned embodiment of the invention can be easily devised by the person skilled in the art, without any undue experimentation from this disclosure; see, e.g., the examples.

In a preferred embodiment of the present invention, the above described methods comprise PCR, ligase chain reaction, restriction digestion, direct sequencing,
Further literature concerning any one of the methods uses and compounds to be employed in accordance with the present invention may be retrieved from public libraries, using for example electronic devices. For example the public database "Medline" may be utilized which is available on Internet, e.g. under http://www.ncbi.nlm.nih.gov/PubMed/medline.html. Further databases and addresses, such as http://www.ncbi.nlm.nih.gov/, http://www.infobiogen.fr/, http://www.fmi.ch/biology/research_tools.html, http://www.tigr.org/, are known to the person skilled in the art and can also be obtained using, e.g., http://www.lycos.com. An overview of patent information in biotechnology and a survey of relevant sources of patent information useful for retrospective searching and for current awareness is given in Berks, TIBTECH 12 (1994), 352-364.

### Brief description of the figures

- **Figure 1:**: Allelic variants of the human CYP2B6 gene. Exons containing mutations resulting in amino acid changes are shown as black boxes, exons resulting in the wt amino acid sequence are shown in white. Base numbering and allele designation was done in accordance with published recommendations for human allele nomenclature (Antonarakis, 1998; http://www.imm.ki.se/CYPalleles/criteria.htm). Only alleles for which definitive haplotype structures could be obtained are shown.
- **Figure 2:**: Primers and novel polymorphisms in the 5'-flanking sequence of CYP2B6 (Sequence Genbank Accession Number AC023172).

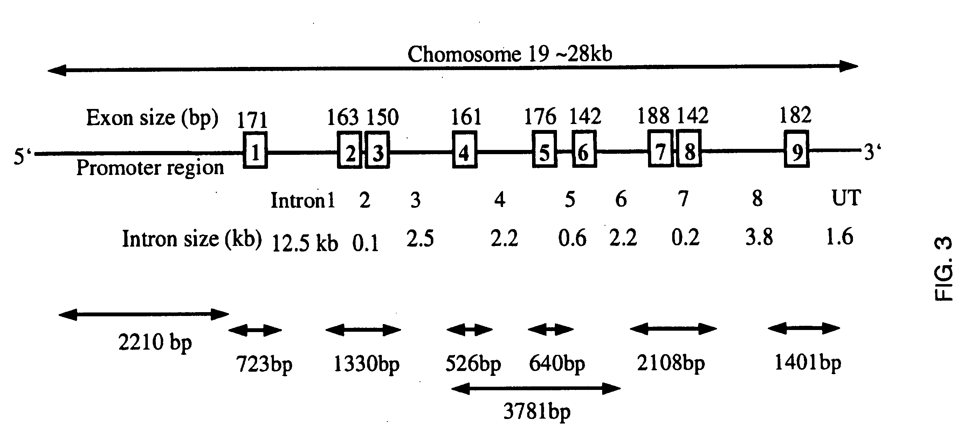

- **Figure 3:**: Human CYP2B6 gene structure and PCR strategy. Exons are shown as boxes with exon size in bp and intron size shown in kb [UT = untranslated region). The position and size of the fragments amplified by PCR are shown underneath. Sequences of primers used for PCR are summarized in Table 1.

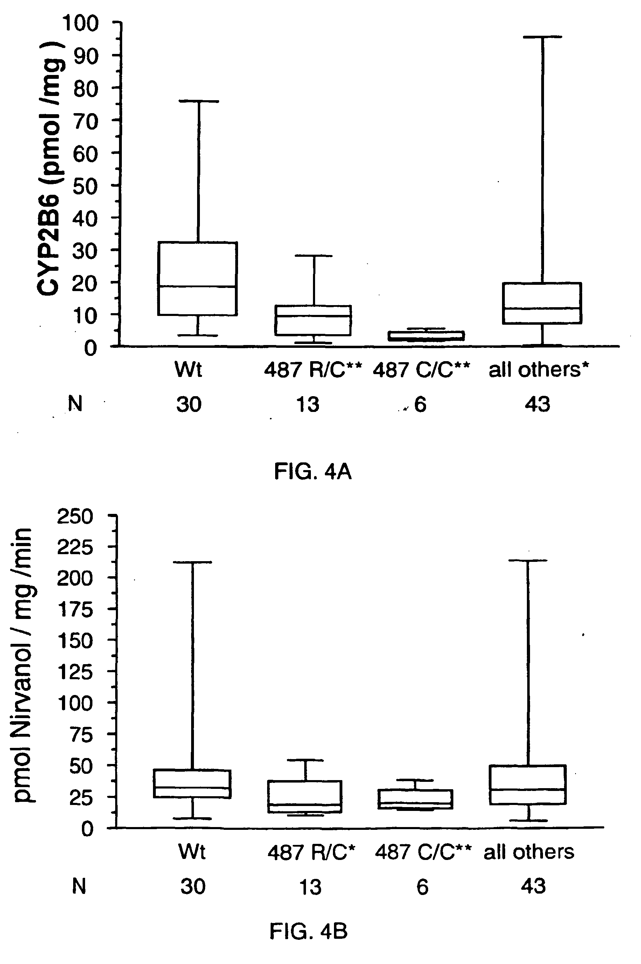

- **Figure 4:**: Analysis of CYP2B6 protein expression (A) and enzymatic function (B). CYP2B6 protein was determined by immunoblotting and the S- mephenytoin N-demethylation to Nirvanol was measured in 92 liver microsome samples. The 5 nonsynonymous CYP2B6 mutations were determined in DNA from the same 92 individuals. Wt indicates the group of 30 individuals with homozygous wild-type genotype; 487 R/C and 487 C/C show the results for individuals heterozygous or homozygous for the exon 9 mutation, respectively. All other mutant genotypes are collectively shown. The average expression and activity of each group is shown as horizontal line. Statistical significance was evaluated by the t-test in comparison to the Wt group: * P < 0.05; ** P < 0.01.

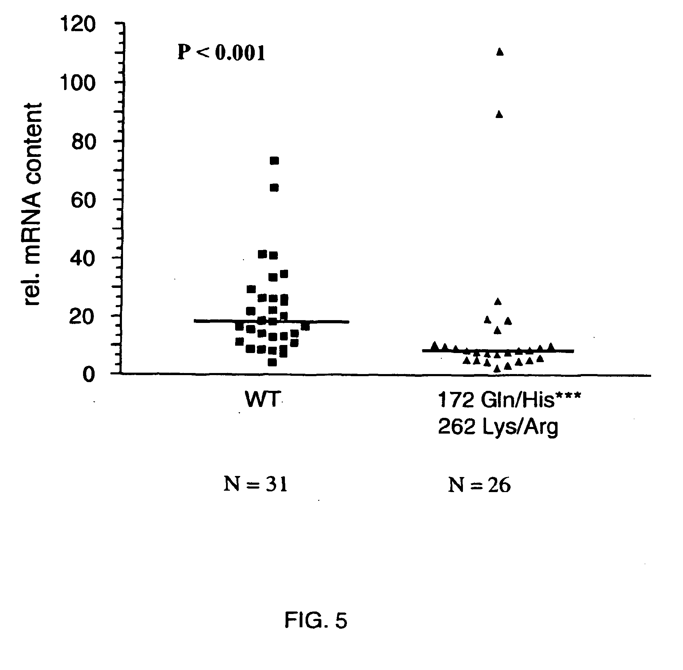

- **Figure 5:**: Analysis of CYP2B6 mRNA expression in human liver. CYP2B6 mRNA was determined by a specific RT-PCR TaqMan assay and measured in 102 liver microsome samples. The 5 nonsynonymous CYP2B6 mutations were determined in DNA from the same 102 individuals. The figure shows the results for the group of 31 individuals homozygous for the wild.type allele (WT) and 26 individuals heterozygous for the 172 and the 262 mutations. The means are shown as horizontal lines. The difference between both groups was statistically significant (Mann-Whitney-test: ***P < 0.001).

The invention will now be described by reference to the following examples which are merely illustrative and are not to be construed as a limitation of the scope of the present invention.

### Examples

### DNA samples and PCR conditions

Leucocyte DNA was isolated by standard methods from 35 German Caucasian individuals. To ensure amplification of CYP2B6 versus the CYP2B7 pseudogene amplification was done with intron-specific and 5'-upstream-specific PCR primer pairs (Table 1). The PCR reactions were performed in a total volume of 50 µl with 100 ng genomic DNA, 50 mM KCI, 10 mM Tris-HCl pH8.3, 1.5 mM MgCl₂, 0.5% Tween 20, 0.01 % Bovine Serum Albumine, 200 µM dNTPs, 2 pMol of each primer and 0.5 U Taq-Polymerase (Promega, Madison). After 5 minutes of denaturation at 95°C, the samples were subjected to the following amplification program: 95°C for 30 s, 50-60°C for 30 s, and 72°C for 30 s - 2 min for 30 cycles with a delayed last step of 10 min at 72°C. Aliquots of each PCR product were subjected to agarose gel electrophoresis to ensure proper amplification. Aliquots of each PCR sample were digested with restriction endonucleases under conditions according to the manufacturers instruction, and the resulting fragments were analyzed on Agarosegel stained with ethidium bromide

### PCR product sequencing

PCR products were directly sequenced using infrared-800 labeled nested primers (MWG Biotech, Ebersberg, Germany) with the Thermo Sequenase fluorescent labelled primer cycle sequencing kit (Amersham Life Sciences, Little Chalfont, England). The nucleotide sequences are shown in Table 1. Sequencing analysis was performed on an automated DNA sequencer (Licor 4200, MWG Biotech, Ebersberg, Germany) using Base ImagIR data collection and image analysis 4.00 software.

### Example 1: Detection of CYP2B6 mutations by PCR-RFLP

### 1.1 C64T mutation in exon 1

PCR reactions were performed in a total volume of 50 µl with 100 ng genomic DNA, 200 µM dNTPs, 2 pmol of primer CYP2B6-1F and primer CYP2B6-1R (Table1), 10 mM Tris-HCl pH 7.0, 50 mM KCI, 1.5 mM MgCl₂, 0.5 % Tween 20, 0.01 % bovine serum albumine and 2.5 U Taq DNA polymerase (Promega Corporation, Madison, USA). After 5 min of denaturation at 95°C the PCR mixtures were subjected to the following conditions : 30 sec 95°C, 30 sec 50 °C and 1 min 72°C for 30 cycles with a delayed last step for 10 min at 72°C in a PTC200 thermal cycler (MJ Research Inc. Watertown, Massachusetts). PCR products were purified with the Quiaquick PCR purification kit (Qiagen, Hilden, Germany). Aliquots of each sample were digested with Hae II (Roche, Basel, Switzerland), and the resulting fragments were analyzed on a 2% Agarosegel (Gibco-BRL, Eggenstein, Germany) stained with ethidium bromide. *Hae* II digestion of wildtype DNA yields fragments of 333 and 390 bp, whereas DNA containing the C64T mutation is not digested and yields an uncleaved fragment of 723 bp.

### 1.2 G516T mutation in exon 4

For amplification of exon 4, the primer pair CYP2B6-4F/-4R was used with the same cycling and analysis conditions as for exon 1, except that 56 °C was used as annealing temperature and the extension step was reduced to 40 seconds. The purified PCR products were digested with restriction enzyme Bsrl (New England Biolabs, Beverly, USA) for 1 hour at 60 °C. Wildtype DNA resulted in three fragments of 241 bp, 268 bp and 17 bp, whereas mutant PCR products resulted in two fragments of 509 bp and 17 bp.

### 1.3 C777A mutation in exon5

Exon 5 was amplified with primers CYP2B6-5F and CYP2B6-5R again with the same cycling conditions as for exon 1 besides that the annealing temperature was 60°C. The mutation could be detected by digestion with restriction enzyme Hae II leaving mutated DNA at postion 777 undigested. The wildtype PCR fragments are 140, 196 and 304 bp, whereas in mutated samples an additional fragment of 500 bp could be detected.

### 1.4 A785G mutation in exon 5

The same PCR fragment as for the C777A mutation could be analyzed for another mutation in exon 5. The A785G mutation destroys a recognition sequence for the restricition enzyme *Sty* I (Roche, Basel, Switzerland). Digestion of wildtype DNA results in fragments of 56, 116, 171 and 297 bp. The A785G mutation creates a fragment of 468 bp. The fragments were analyzed in a 2.5 % MetaPhor Agarosegel (FMC, Rockland, USA) where fragments 56 and 116 could not be resolved.

### 1.5 C1459T mutation in exon 9

Exon 9 was amplified with primers CYP2B6-9F and primer CYP2B6-9R using the same conditions as for exon 1 with the exception of a prolonged extension step of 1 min 30 sec and a raised annealing step at 60°C. The C1459T mutation introduces a restriction enzyme site for *Bgl* II (Roche, Basel, Switzerland) which yields fragments of 216 and 1185 bp. Wildtype DNA yields an uncleaved PCR fragment of 1401 bp. The fragments were separated in a 1.5% Agarosegel and could be detected after ethidium bromide staining.

### Example 2: Polymorphisms in the CYP2B6 5'-flanking sequence (PCR-RFLP) and functional analysis of CYP2B6 polymorphisms in a human liver bank

A specific direct DNA sequencing strategy was developed to amplify 2.5 kb of the *CYP2B6* 5'-flanking region. Figure 2 shows the 5'-flanking sequence and the location of the primers used for its specific amplification and sequencing (see also Table 1). Five novel mutations were identified by complete sequence analysis of DNA from 35 individuals. These are: T-82C, T-750C, C-1185G, T-1455C, A-1777G (Table 2). The frequencies of these mutations varied between 1.4 and 54.2 % (Table 2).

### Example 3: Description of the results

Genomic sequences of the human CYP2B locus were used which were elucidated in the course of the human genome project and made publically available through the internet http://www-bio.llnl.gov/genome/genome.html (Annotation: Click on "Genomic sequencing of regions of chr 19", and enter the clone id22376 in the query box at the bottom of the form. This take you to the tiling path, with the list (ordered from centromere to telomere) of clones sequenced, and links to their sequence and accessions (when available) in Genbank. The cosmid F22376 spans the entire CYP2B6 gene and is currently sequenced but is still in the annotation stage.) to design oligonucleotide primers to amplify genomic DNA containing the CYP2B6 coding regions (Table 1). It was shown by sequence comparison that the amplified segments had been specifically derived from the CYP2B6 gene and not from CYP2B7 in all cases.
To search for polymorphism, genomic DNA from 35 randomly selected, healthy Caucasian individuals was amplified with all CYP2B6-specific primer pairs and the amplified fragments were completely sequenced in both directions. A total of five non-synonymous mutations were detected in exons 1, 4, 5 and 9, which occured with different frequencies and in different heterozygous and homozygous combinations (Table 2). Linkage analysis resulted in the elucidation of 7 different alleles summarized in Table 3 and Figure 1. Analysis of the data obtained from the 35 completely sequenced individuals revealed a total of 6 mutant alleles in addition to the wild- type allel (Table 3).
Diagnostic tests were developed to easily detect each of the five mutations in genomic DNA. Each Mutation could be shown to either abolish or create an enzymatic restriction site (Table 2). It was thus possible in all cases to develop an assay based on 1) *CYP2B6*-specific amplification of the gene fragment that contains the mutation and 2) digestion with a suitable restriction enzyme. The frequency of each mutation was estimated in a representative German population using the dignostic test designed for it (Table 2).

### Example 4: Analysis of the functional significance of the CYP2B6 mutations

The functional significance of the mutations in the CYP2B6 gene (Table 2) was investigated by analyzing expression and enzymatic activity of CYP2B6 in a large human liver bank. A specific monoclonal antibody, MAB-2B6 (Gentest Corp., Woburn, MA, USA) was used to immunoquantitate microsomal CYP2B6. *S-*mephenytoin *N*-demethylation to Nirvanol was measured as a rather CYP2B6-specific pathway. The five nonsynonymous mutations were determined in all samples. Compared to the homozygous wild-types, who expressed on average 23.5 ± 16.3 pmol/mg CYP2B6, significantly reduced levels were measured for carriers of the C1459T (R487C) mutation (alleles **5* and **7*): heterozygotes expressed 9.7 ± 7.5 pmol/mg (P = 0.006, N=13) and homozygotes only 3.05 ± 1.4 pmol/mg (P = 0.005, N=6, *t*-test; Fig. 4A). Thus, compared to the homozygous wildtype, CYP2B6 protein expression of homozygous carriers of the R487C mutation in exon 9 was almost eightfold reduced. The mean enzyme activities of heterozygous (25.9 ± 15.2 pmoles of nirvanol formed/min*mg, P = 0.02, *t*-test) and homozygous carriers of R487C (22.2 ± 8.4, P = 0.008, *t*- test) were also significantly reduced compared to the Wt group (49.2 ± 49.4; Fig. 4B).

A significant difference in protein expression was also found for individuals with other mutation than the exon 9 mutation, who expressed 16.9 ± 18 pmol/mg, which was significantly less compared to the homozygous wild-type (P=0.016; Figure 4A).

Finally, quantitation of CYP2B6 mRNA transcripts was performed using a newly developed real-time PCR assay in total RNA prepared from human livers. As shown in Fig. 5, there was a significant difference in mRNA expression between the homozygous wild-types and the group of individuals being heterozygous for the 172 Gln>His and the 262 Lys>Arg mutations, who expressed only about 50 % of the transcripts (P<0.001). The decrease in mRNA expression is also influenced by linkage to the promoter mutation T-750C, which is present at about 54 % and which affects a putative hepatic nuclear factor 1 (hNF1) consensus binding site.

### Example 5: Significance of the results

Genetic polymorphisms in drug metabolizing enzymes have been shown to be one of the major factors of interindividual variations in drug response. Genetic polymorphisms may affect gene expression, mRNA and protein stability, enzyme activity and/or substrate specificity for a given gene product and thus leads to complex variations in the way the organism acts on the drug. The precise consequences a mutation may have depends on the drug in question, e.g. whether it is a prodrug or not, or whether the metabolites formed are themselves active or toxic.
A number of clinically important drugs have been shown to be substrates for CYP2B6, including the anticancer prodrugs cyclophosphamide and ifosfamide, the antioestrogenic tamoxifen, the platelet aggregation inactivator, clopidogrel, but also addictive drugs including nicotine and ecstasy (Table 4).

CYP2B6 is involved in the metabolic activation and inactivation of a number of clinically important drugs such as the frequently used antineoplastic agents cyclophosphamide and ifosfamide, the antioestrogen tamoxifen, the newer platelet aggregation inhibitor Clopidogrel, but also environmental toxicants and recreational drugs such as nicotine and ecstasy (Table 4).

Through genomic sequence analysis, evidence was obtained for a total of 7 distinct alleles which contain between one and three amino acid mutations. Some of these alleles occur with high frequency in the normal population. The biochemical properties of the CYP2B6 protein such as protein stability, activity, or substrate specificity are changed by these mutations and will lead to interindividual differences in drug metabolism.

The use of diagnostic tests for the newly identified mutations will help to predict therapeutic efficacy and/or drug toxicity related to drugs metabolized by CYP2B6. In addition, since CYP2B6 was also shown to participate in nicotine metabolism, *CYP2B6* genetic diagnosis will help to predict smoking habits (Pianezza, Nature 393 (1998), 750).

**Table 1A: Sequences of primers used for PCR amplification of CYP2B6 coding exons**

| **Primer** **designation** | **5'-3' sequence** | **Primer** **location** | **Amplified** **exon** | **Product** **size (bp)** |
|---|---|---|---|---|
| CYP2B6-1F | ACATTCACTTGCTCACCT | 5' UT | 1 | 723 |
| CYP2B6-1R | GTAAATACCACTTGACCA | Intron 1 | | |
| CYP2B6-2F | ATCCTACTCAGAATGATGCACAAC | Intron 1 | 2+3 | 1330 |
| CYP2B6-3R | ATTACAGGTGAGAGTCATCACATC | Intron 3 | | |
| CYP2B6-4F | GGTCTGCCCATCTATAAAC | Intron 3 | 4 | 526 |
| CYP2B6-4R | CTGATTCTTCACATGTCTGCG | Intron 4 | | |
| CYP2B6-4F1 | TCCCTGGGATTTAACTGTACTCAC | Intron 3 | 4+5+6 | 3781 |
| CYP2B6-6R | CAGAATTGGCTTGGTTGGAATCTA | Intron 7 | | |
| CYP2B6-5F | GACAGAAGGATGAGGGAGGAA | Intron 4 | 5 | 640 |
| CYP2B6-5R | CTCCCTCTGTCTTTCATTCTGT | Intron 5 | | |
| CYP2B6-7F | GTGATTATTCATTAATTGGGTTC | Intron 6 | 7+8 | 2108 |
| CYP2B6-8R | TGCAATGGTTGATTGATGCTC | Intron 8 | | |
| CYP2B6-9F | TGAGAATCAGTGGAAGCCATAGA | Intron 8 | 9 | 1401 |
| CYP2B6-9R | | 3' UT | | |

**Table 1B: Sequences of primers used for PCR amplification of CYP2B6 promoter region**

| **Primer** **designation** | **5'-3' sequence** | **Primer** **location** | **Product** **size (bp)** |
|---|---|---|---|
| CYP2B6-P1F | ACATTCACTTGCTCACCT | 5' upstream | |
| CYP2B6-P1R | GTAAATACCACTTGACCA | Intron 1 | 723 |
| CYP2B6-P2F | TGCCGACATGTGATGTCT | 5' upstream | 2210 |
| CYP2B6-P2R | AGGTGAGCAAGTGAATGT | 5' upstream | |

**Table 1C: Sequences of primers used for sequencing of CYP2B6 coding exons**

| **Sequence** **primer** **designation** | **5'-3'sequence** | **Primer** **location** | **Exon** |
|---|---|---|---|
| seqCYP2B6-1F | ATAACAGGGTGCAGAGGCA | 5' UT | 1 |
| seqCYP2B6-1R | AAGTACCAAGGCAAGAAGCA | Intron 1 | 1 |
| seqCYP2B6-2F | GGCTAATTACCAATCTGGT | Intron 2 | 2 |
| seqCYP2B6-2R | ATATACTCCCTTCCCTGATGCA | Intron 3 | 2 |
| seqCYP2B6-3F | ACTCAGAGCCTTCTTCCAACT | Intron 3 | 3 |
| seqCYP2B6-3R | ACCTGCATCTCTCAGTGTTTCATT | Intron 4 | 3 |
| seqCYP2B6-4F | TAGGTGACAGCCTGATGTTC | Intron 4 | 4 |
| seqCYP2B6-4R | TCATCCTTTTCTCGTGTGTTCT | Intron 5 | 4 |
| seqCYP2B6-5F | TCTCTCCCTGTGACCTGCTAGCT | Intron 5 | 5 |
| seqCYP2B6-5R | TCTTCTCACCTCTCCATCTT | Intron 6 | 5 |
| seqCYP2B6-6F | TATACACAGCAAGGCTACAG | Intron 6 | 6 |
| seqCYP2B6-6R | ATTTCTGCAGCTCAGAAGGA | Intron 7 | 6 |
| seqCYP2B6-7F | GATTACAGGCATGAGCCACCAT | Intron 7 | 7 |
| seqCYP2B6-/R | ATTAAGAGAATCCAGGATGCC | Intron 8 | 7 |
| seqCYP2B6-8F | TTTTGTGGAGTGTGTGGAGGGT | Intron 8 | 8 |
| seqCYP2B6-8R | TTCTCAAGTTGGGGATAGTA | Intron 9 | 8 |
| seqCYP2B6-9F | AGAGCGAAGTGTATGCACCT | Intron 9 | 9 |
| seqCYP2B6-9R | AGAGCCATTGTCTACAGAGG | 3' UT | 9 |

**Table 1D: Sequences of primers used for sequencing CYP2B6 promoter region**

| **Sequence** **primer** **designation** | **5'-3'sequence** | **Primer** **location** |
|---|---|---|
| seqCYP2B6-P1F | ACAATACCTCACTAAGAGTG | 5' upstream |
| seqCYP2B6-P2F | GTCTCAGTTTCTGTCTCCTTC | 5' upstream |
| seqCYP2B6-P3F | TGAACATGCACTACCACCA | 5' upstream |
| seqCYP2B6-P4R | ATCCTTGCATGTGTATGAGC | 5' upstream |
| seqCYP2B6-P5R | TGAACCAGGAGTAGCAAGAG | Exon1 |

**Table 2: Total frequencies of CYP2B6 mutations^{a}**

| **Nucleotide change** | **Predicted** **consequence** | **Location** | **RFLP** | **Frequency** **(%)** |
|---|---|---|---|---|
| A-1777G | unknown | 5' upstream | *BceF* I created | 8.6 |
| T-1455C | unknown | 5' upstream | *Acc* I abolished | 31.4 |
| C-1185G | unknown | 5' upstream | *Ava* II created | 5.7 |
| T-750C | unknown | 5' upstream | *Mse* I abolished | 54.2 |
| T-82C | unknown | 5' upstream | no sites found | 1.4 |
| C64T | Arg22Cys | Exon 1 | *Hae* II abolished | 5.3 |
| G516T | Gln172His | Exon 4 | *Bsr* I abolished | 28.6 |
| C777A | Ser259Arg | Exon 5 | *Hae* II abolished | 0.5 |
| A785G | Lys262Arg | Exon 5 | *Sty* I abolished | 32.6 |
| C1459T | Arg487Cys | Exon 9 | *Bgl* II created | 14.0 |
| C78T | silent | Exon 1 | *Bsp*H I created | 2.9 |
| G216C | silent | Exon 2 | *EcoR* II created | 7.1 |
| G714A | silent | Exon 5 | *Pst* I abolished | 1.4 |
| C732T | silent | Exon 5 | no sites found | 2.9 |
| A59T | unknown | Intron 2 | - | 7.1 |
| C-18T | unknown | Intron 3 | - | 21.4 |

| | | | | |
|---|---|---|---|---|
| ^{a} Frequencies were determined in 215 individuals by using PCR-RFLP tests for nonsynonymous mutations and by sequencing in 35 individuals. | | | | |

**Table 3: Alleles of CYP2B6**

| **Allele** | **Nucleotide changes** | **Amino acid mutations** |
|---|---|---|
| *CYP2B6*1* | none | |
| *CYP2B6*2* | C64T | Arg22Cys |
| *CYP2B6*3* | C777A | Ser259Arg |
| *CYP2B6*4* | A785G | Lys262Arg |
| *CYP2B6*5* | C1459T | Arg487Cys |
| *CYP2B6***6* | G516T; A785G | Gln172His; Lys262Arg |
| *CYP2B6*7* | G516T; A785G; C1459T | Gln172His; Lys262Arg; Arg487Cys |

**Table 4: Substrates for CYP2B6**

| **Substrate** | **Type** | **Reaction catalyzed** | **Reference** |
|---|---|---|---|
| Cyclophosphamide | Anti-cancer drug | Aliphatic hydroxylation, N-dealkylation | Chang et al 1993 |
| Iphosphamide | Anti-cancer drug | Aliphatic hydroxylation, N-dealkylation | Granvil et al., 1999 |
| S-Mephenytoin | Anticonvulsant | N-Demethylation | Heyn et al., 1996 |
| Tamoxifen | Anti-oestrogen | Hydroxylation | Gillam et al., 1999 |
| RP 73401 | | | Stevens et al., 1997 |
| Clopidogrel | Platelet aggregation inhibitor | Hydroxylation | Coukell and Markham, 1997 |
| Nicotine | Major tobacco constituent | C-oxidation | Flammang et al., 1992 |
| MDMA ("ecstasy") and MDEA ("eve") | recreational drugs | N-dealkylation | |
| Dibenzo-[*a,h*]-anthracene | Precarcinogen | | Shou et al., 1996 |
| 6-Aminochrysene | Precarcinogen | | Mimura et al., 1993 |

### References

Ariyoshi N, Oguri K, Koga N, Yoshimura H, Funae Y. Metabolism of highly persistent PCB congener, 2,4,5,2',4',5'-hexachlorobiphenyl, by human CYP2B6. Biochem Biophys Res Commun 1995 Jul 17; 212(2): 455-60
Carrano AV, Lamerdin J, Ashworth LK, Watkins B, Branscomb E, Slezak T, Raff M, de Jong PJ, Keith D, McBride L, et al.. A high-resolution, fluorescence-based, semiautomated method for DNA fingerprinting. Genomics 1989 Feb; 4(2): 129-36
Chang TK, Weber GF, Crespi CL, Waxman DJ. Differential activation of cyclophosphamide and ifosphamide by cytochromes P-450 2B and 3A in human liver microsomes. Cancer Res 1993 Dec 1; 53(23): 5629-37
Coukell AJ, Markham A., Clopidogrel. Drugs 1997: 54, 745 - 750
Gervot L, Rochat B, Gautier JC, Bohnenstengel F, Kroemer H, de Berardinis V, Martin H, Beaune P, de Waziers I. Human CYP2B6: expression, inducibility and catalytic activities. Pharmacogenetics 1999 Jun; 9(3): 295-306
Code EL, Crespi CL, Penman BW, Gonzalez FJ, Chang TK, Waxman DJ. Human cytochrome P4502B6: interindividual hepatic expression, substrate specificity, and role in procarcinogen activation. Drug Metab Dispos 1997 Aug; 25(8): 985-93
Gonzalez FJ, Crespi CL, Czerwinski M, Gelboin HV. Analysis of human cytochrome P450 catalytic activities and expression. Tohoku J Exp Med 1992 Oct; 168(2): 67-72
Granvil CP, Madan A, Sharkawi M, Parkinson A, Wainer IW. Role of CYP2B6 and CYP3A4 in the in vitro N-dechloroethylation of (R)- and (S)-ifosfamide in human liver microsomes. Drug Metab Dispos 1999 Apr; 27(4): 533-41
Hoffman SM, Fernandez-Salguero P, Gonzalez FJ, Mohrenweiser HW. Organization and evolution of the cytochrome P450 CYP2A-2B-2F subfamily gene cluster on human chromosome 19. J Mol Evol 1995 Dec; 41(6): 894-900 Kobayashi K, Abe S, Nakajima M, Shimada N, Tani M, Chiba K, Yamamoto T. Role of human CYP2B6 in S-mephobarbital N-demethylation. Drug Metab Dispos 1999 Dec; 27(12): 1429-33
Klaus-Peter Kreth, Karl-Artur Kovar, Matthias Schwab, Ulrich M. Zanger. Identification of the Human Cytochromes P450 involved in the Oxidative metabolism of "Ecstasy"-Related Designer-Drugs. Biochemical Pharmacology 2000 (in press)
Miles J:S., McLaren A.W., Wolf C.R.. A novel human cytochrome P450 gene (P450IIB): chromosomal localization and evidence for alternative splicing. Nucleic Acids Res. 16:5783-5795 (1987).
Miles,J.S., Spurr,N.K., Gough,A.C., Jowett,T., McLaren,A.W., Brook,J.D. and Wolf,C.R. A novel human cytochrome P450 gene (P450IIB): chromosomal localization and evidence for alternative splicing. Nucleic Acids Res. 16 (13), 5783-5795 (1988)
Miles J.S., McLaren A.W., Wolf C.R.. Alternative splicing in the human cytochrome P45011 B6 gene generates a high level of aberrant messages. Nucleic Acids Res. 17: 8241-8255 (1989).
Mimura M, Baba T, Yamazaki H, Ohmori S, Inui Y, Gonzalez FJ, Guengerich FP, Shimada T. Characterization of cytochrome P-450 2B6 in human liver microsomes. Drug Metab Dispos 1993 Nov-Dec; 21(6): 1048-56
Shou M, Krausz KW, Gonzalez FJ, Gelboin HV. Metabolic activation of the potent carcinogen dibenzo[a,h]anthracene by cDNA-expressed human cytochromes P450. Arch Biochem Biophys 1996 Apr 1;328(1):201-7
Stevens JC, White RB, Hsu SH, Martinet M. Human liver CYP2B6-catalyzed hydroxylation of RP 73401. J Pharmacol Exp Ther 1997 Sep; 282(3): 1389-95
Yamano, S., Nhamburo, P.T., Aoyama, T., Meyer, U.A., Inaba, T., Kalow, W., Gelboin, H.V., McBride, O.W. and Gonzalez, F.J. cDNA cloning and sequence and cDNA-directed expression of human P450 IIB1: identification of a normal and two variant cDNAs derived from the CYP2B locus on chromosome 19 and differential expression of the IIB mRNAs in human liver. Biochemistry 28 (18), 7340-7348 (1989)
Ekins, S., VandenBranden M, Ring, B.J., Wrighton, S.A., Eamination of puported probes of human CYP2B6, Pharmacogenetics 1997 Jun:7(3): 165-79
Heyn H., White, R.B., Stevens, J.C., Catalytic role of cytochrome P4502B6 in the N-demethylation of S-mephnytoin Drug Metab. Dispos. 24(9), 948-954 (1996)
Kobayashi K., Abe, S., Nakajima, M., Shimada, N., Tani, M., Chiba, K., Yamamoto Role of human CYP2B6 in S-mephobarbital N-demethylation. Drug Metab. Dispos. 27(12), 1429-1433 (1999)
Kumar, G.N., Dykstra, J., Roberts, E.M., Jayanti, V.K., Hickman, D., Uchic, J., Yao, Y., Surber B., Thomas, S., Granneman, G.R., Potent inhibition of the cytochrome P-450 3A-mediated human liver microsomal metabolism of a novel HIV protease inibitor by ritonavir: A positive drug-drug interaction, Drug Metab. Dispos. 27(8), 902-908 (1999)
Yanev, S., Kent, U.M., Pandova, B., Hollenberg, P.F., Selective mechanism-based inactivation of cytochromes P-450 2B1 and P-450 2B6 by a series of xanthates, Drug Metab. Dispos. 27(5), 600-604 (1999)

## Claims

1. A method of diagnosing drug hypersensitivity related to the presence of a molecular variant of the CYP2B6 gene comprising determining in a sample from a subject the presence of a polynucleotide selected from the group consisting of:
(a) a polynucleotide encoding a CYP2B6 polypeptide or fragment thereof, wherein said polypeptide comprises an amino acid substitution of Gln172 to His in exon 4 and/or Arg487 to Cys in exon 9 of the CYP2B6 polypeptide having accession No: M29874; and
(b) a polynucleotide capable of hydridizing to the CYP2B6 gene, wherein said polynucleotide is having a T at a position corresponding to position 516 or 1459 of the CYP2B6 gene having accession No: M29874, wherein nucleotide A at position 7 has been numbered +1;
wherein the presence of said polynucleotide or fragment thereof is considered indicative of said drug hypersensitivity in said method.

2. The method of claim 1 comprising PCR, ligase chain reaction, restriction digestion, direct sequencing, nucleic acid amplification techniques, hydridization techniques or immunoassays.

3. Use of an oligo- or polynucleotide for diagnosing drug hypersensitivity related to the presence of a molecular variant of the CYP2B6 gene comprising determining in a sample from a subject the presence of a polynucleotide selected from the group consisting of:
(a) a polynucleotide encoding a CYP2B6 polypeptide or fragment thereof, wherein said polypeptide comprises an amino acid substitution of Gln172 to His in exon 4 and/or Arg487 to Cys in exon 9 of the CYP2B6 polypeptide having accession No: M29874; and
(b) a polynucleotide capable of hybridizing to the CYP2B6 gene, wherein said polynucleotide is having a T a position corresponding to position 516 or 1459 of the CYP2B6 gene having accession No: M29874, wherein nucleotide A at position 7 has been numbered +1.

4. An in vitro method for the development of a pharmacodynamic profile of drugs that are substrates of the CYP2B6 gene product for a patient or for improving the therapy of disorders with drugs that are targets of the CYP2B6 gene product comprising using the CYP2B6 gene single nucleotide polymorphism at position 516, or 1459 as defined in claim 1.

5. The method of claim 4, wherein said disorder is cancer.

## Patentansprüche

1. Verfahren zur Diagnose von Arzneimittelüberempfindlichkeit, die im Bezug steht mit dem Vorhandensein einer molekularen Variante des CYP2B6-Gens, umfassend Bestimmen des Vorhandenseins eines Polynukleotids in einer Probe eines Subjekts, wobei das Polynukleotid ausgewählt ist aus der Gruppe bestehend aus:
(a) einem für ein CYP2B6-Polypeptid oder ein Fragment davon codierenden Polynukleotid, wobei das Polypeptid eine Aminosäuresubstitution von Gln172 zu His in Exon 4 umfasst und/oder von Arg487 zu Cys in Exon 9 des CYP2B6-Polypeptids mit der Accession-No. M29874; und
(b) einem Polynukleotid, das mit dem CYP2B6-Gen hybridisieren kann, wobei das Polynukleotid ein T an einer Position aufweist, die der Position 516 oder 1459 des CYP2B6-Gens mit der Accession-No. M29874 entspricht, wobei Nukleotid A an Position 7 mit +1 nummeriert wurde;
wobei das Vorhandensein des Polynukleotids oder Fragments davon als Hinweis für die Arzneimittelüberempfindlichkeit in diesem Verfahren angesehen wird.

2. Verfahren nach Anspruch 1, umfassend PCR, Ligase-Kettenreaktion, Restriktionsverdauung, direktes Sequenzieren, Nukleinsäureamplifikationstechniken, Hybridisierungstechniken oder Immunoassays.

3. Verwendung von Oligo- bzw. Polynukleotiden zur Diagnose von Arzneimittelüberempfindlichkeit, die im Bezug steht mit dem Vorhandensein einer molekularen Variante des CYP2B6-Gens, umfassend Bestimmen des Vorhandenseins eines Polynukleotids in einer Probe eines Subjekts, wobei das Polynukleotid ausgewählt ist aus der Gruppe bestehend aus:
(a) einem für ein CYP2B6-Polypeptid oder ein Fragment davon codierenden Polynukleotid, wobei das Polypeptid eine Aminosäuresubstitution von Gln172 zu His in Exon 4 umfasst und/oder von Arg487 zu Cys in Exon 9 des CYP2B6-Polypeptids mit der Accession-No. M29874; und
(b) einem Polynukleotid, das mit dem CYP2B6-Gen hybridisieren kann, wobei das Polynukleotid ein T an einer Position aufweist, die der Position 516 oder 1459 des CYP2B6-Gens mit der Accession-No. M29874 entspricht, wobei Nukleotid A an Position 7 mit +1 nummeriert wurde.

4. In-vitro-Verfahren zur Entwicklung eines pharmako-dynamischen Profils von Arzneimitteln für einen Patient, die Substrate des CYP2B6-Genprodukts sind, oder zur Verbesserung der Behandlung von Erkrankungen mit Arzneimitteln, die Ziel des CYP2B6-Genprodukts sind, umfassend Verwenden des CYP2B6-Gen Einzelnukleotidpolymorphismus an Position 516 oder 1459, wie in Anspruch 1 definiert.

5. Verfahren nach Anspruch 4, wobei die Erkrankung Krebs ist.

## Revendications

1. Méthode de diagnostic d'hypersensibilité aux médicaments en rapport avec la présence d'une variante moléculaire du gène CYP2B6, comprenant la détermination dans un échantillon d'un sujet de la présence d'un polynucléotide sélectionné dans le groupe constitué de:
(a) un polynucléotide codant un polypeptide CYP2B6 ou un fragment de ce dernier, dans lequel ledit polypeptide comprend une substitution d'acide aminé de Gln172 à His en exon 4 et/ou Arg487 à Cys en exon 9 du polypeptide CYP2B6 avec le n° d'accès M29874; et
(b) un polynucléotide capable de s'hybrider au gène CYP2B6, dans lequel ledit polynucléotide présente un T à une position correspondent à la position 516 ou 1459 du gène CYP2B6 ayant le n° d'accès M29874, dans lequel le nucléotide A à la position 7 a été numéroté +1;
la présence dudit polynucléotide ou fragment de ce dernier étant considérée comme indicatrice de ladite hypersensibilité aux médicaments dans ladite méthode.

2. Méthode selon la revendication 1, comprenant un PCR, une réaction de ligation en chaîne, une digestion par des enzymes de restriction, un séquençage direct, des techniques d'amplification d'acide nucléique, des techniques d'hybridation ou des immunoessais.

3. Utilisation d'un oligonucléotide ou polynucléotide pour le diagnostic d'hypersensibilité aux médicaments en rapport avec la présence d'une variante moléculaire du gène CYP2B6, comprenant la détermination dans un échantillon d'un sujet de la présence d'un polynucléotide sélectionné dans le groupe constitué de:
(a) un polynucléotide codant un polypeptide CYP2B6 ou un fragment de ce dernier, dans lequel ledit polypeptide comprend une substitution d'acide aminé de Gln172 à His en exon 4 et/ou Arg487 à Cys en exon 9 du polypeptide CYP2B6 avec le n° d'accès M29874; et
(b) un polynucléotide capable de s'hybrider au gène CYP2B6, dans lequel ledit polynucléotide présente un T à une position correspondent à la position 516 ou 1459 du gène CYP2B6 ayant le n° d'accès M29874, dans lequel le nucléotide A à la position 7 a été numéroté +1.

4. Méthode in vitro pour le développement d'un profile pharmacodynamique de médicaments pour un patient qui sont des substrats du produit génétique CYP2B6 ou pour l'amélioration de la thérapie de troubles avec des médicaments qui sont des cibles du produit génétique CYP2B6, comprenant l'utilisation du polymorphisme nucléotidique simple du gène à la position 516 ou 1459 comme défini à la revendication 1.

5. Méthode selon la revendication 4, dans laquelle ledit trouble est un cancer.


### SEQUENCE LISTING

<110> Epidauros Biotechnologie AG
<120> Polymorphims in the cytochrome P450 2B6 (CYP2B6) gene and uses thereof in diagnostic and therapeutic applications
<130> D 2762 PCT
<140>
   <141>
<160> 64
<170> PatentIn Ver. 2.1
<210> 1
   <211> 18
   <212> DNA
   <213> Artificial Sequence
<220>
   <223> Description of Artificial Sequence: artificial sequence
<400> 1
   acattcactt gctcacct 18
<210> 2
   <211> 18
   <212> DNA
   <213> Artificial Sequence
<220>
   <223> Description of Artificial Sequence: artificial sequence
<400> 2
   gtaaatacca cttgacca 18
<210> 3
   <211> 24
   <212> DNA
   <213> Artificial Sequence
<220>
   <223> Description of Artificial Sequence: artificial sequence
<400> 3
   atcctactca gaatgatgca caac 24
<210> 4
   <211> 24
   <212> DNA
   <213> Artificial Sequence
<220>
   <223> Description of Artificial Sequence: artificial sequence
<400> 4
   attacaggtg agagtcatca catc 24
<210> 5
   <211> 24
   <212> DNA
   <213> Artificial Sequence
<220>
   <223> Description of Artificial Sequence: artificial sequence
<400> 5
   ggtctgccca tctataaac 19
<210> 6
   <211> 24
   <212> DNA
   <213> Artificial Sequence
<220>
   <223> Description of Artificial Sequence: artificial sequence
<400> 6
   ctgattcttc acatgtctgcg 21
<210> 7
   <211> 24
   <212> DNA
   <213> Artificial Sequence
<220>
   <223> Description of Artificial Sequence: artificial sequence
<400> 7
   tccctgggat ttaactgtac tcac 24
<210> 8
   <211> 24
   <212> DNA
   <213> Artificial Sequence
<220>
   <223> Description of Artificial Sequence: artificial sequence
<400> 8
   cagaattggc ttggttggaa tcta 24
<210> 9
   <211> 21
   <212> DNA
   <213> Artificial Sequence
<220>
   <223> Description of Artificial Sequence: artificial sequence
<400> 9
   gacagaagga tgagggagga a 21
<210> 10
   <211> 22
   <212> DNA
   <213> Artificial Sequence
<220>
   <223> Description of Artificial Sequence: artificial sequence
<400> 10
   ctccctctgt ctttcattct gt 22
<210> 11
   <211> 23
   <212> DNA
   <213> Artificial Sequence
<220>
   <223> Description of Artificial Sequence: artificial sequence
<400> 11
   gtgattattc attaattggg ttc 23
<210> 12
   <211> 20
   <212> DNA
   <213> Artificial Sequence
<220>
   <223> Description of Artificial Sequence: artificial sequence
<400> 12
   tgcaatggtt gattgatgct c 21
<210> 13
   <211> 23
   <212> DNA
   <213> Artificial Sequence
<220>
   <223> Description of Artificial Sequence: artificial sequence
<400> 13
   tgagaatcag tggaagccat aga 23
<210> 14
   <211> 25
   <212> DNA
   <213> Artificial Sequence
<220>
   <223> Description of Artificial Sequence: artificial sequence
<400> 14
   taattttcga taatctcact cctgc 25
<210> 15
   <211> 19
   <212> DNA
   <213> Artificial Sequence
<220>
   <223> Description of Artificial Sequence: artificial sequence
<400> 15
   ataacagggt gcagaggca 19
<210> 16
   <211> 20
   <212> DNA
   <213> Artificial Sequence
<220>
   <223> Description of Artificial Sequence: artificial sequence
<400> 16
   aagtaccaag gcaagaagca 20
<210> 17
   <211> 19
   <212> DNA
   <213> Artificial Sequence
<220>
   <223> Description of Artificial Sequence: artificial sequence
<400> 17
   ggctaattac caatctggt 19
<210> 18
   <211> 22
   <212> DNA
   <213> Artificial Sequence
<220>
   <223> Description of Artificial Sequence: artificial sequence
<400> 18
   atatactccc ttccctgatg ca 22
<210> 19
   <211> 21
   <212> DNA
   <213> Artificial Sequence
<220>
   <223> Description of Artificial Sequence: artificial sequence
<400> 19
   actcagagcc ttcttccaac t 21
<210> 20
   <211> 24
   <212> DNA
   <213> Artificial Sequence
<220>
   <223> Description of Artificial Sequence: artificial sequence
<400> 20
   acctgcatct ctcagtgttt catt 24
<210> 21
   <211> 20
   <212> DNA
   <213> Artificial Sequence
<220>
   <223> Description of Artificial Sequence: artificial sequence
<400> 21
   taggtgacag cctgatgttc 20
<210> 22
   <211> 22
   <212> DNA
   <213> Artificial Sequence
<220>
   <223> Description of Artificial Sequence: artificial sequence
<400> 22
   tcatcctttt ctcgtgtgtt ct 22
<210> 23
   <211> 23
   <212> DNA
   <213> Artificial Sequence
<220>
   <223> Description of Artificial Sequence: artificial sequence
<400> 23
   tctctccctg tgacctgcta gct 23
<210> 24
   <211> 20
   <212> DNA
   <213> Artificial Sequence
<220>
   <223> Description of Artificial Sequence: artificial sequence
<400> 24
   tcttctcacc tctccatctt 20
<210> 25
   <211> 20
   <212> DNA
   <213> Artificial Sequence
<220>
   <223> Description of Artificial Sequence: artificial sequence
<400> 25
   tatacacagc aaggctacag 20
<210> 26
   <211> 20
   <212> DNA
   <213> Artificial Sequence
<220>
   <223> Description of Artificial Sequence: artificial sequence
<400> 26
   atttctgcag ctcagaagga 20
<210> 27
   <211> 22
   <212> DNA
   <213> Artificial Sequence
<220>
   <223> Description of Artificial Sequence: artificial sequence
<400> 27
   gattacaggc atgagccacc at 22
<210> 28
   <211> 21
   <212> DNA
   <213> Artificial Sequence
<220>
   <223> Description of Artificial Sequence: artificial sequence
<400> 28
   attaagagaa tccaggatgc c 21
<210> 29
   <211> 22
   <212> DNA
   <213> Artificial Sequence
<220>
   <223> Description of Artificial Sequence: artificial sequence
<400> 29
   ttttgtggag tgtgtggagg gt 22
<210> 30
   <211> 20
   <212> DNA
   <213> Artificial Sequence
<220>
   <223> Description of Artificial Sequence: artificial sequence
<400> 30
   ttctcaagtt ggggatagta 20
<210> 31
   <211> 20
   <212> DNA
   <213> Artificial Sequence
<220>
   <223> Description of Artificial Sequence: artificial sequence
<400> 31
   agagcgaagt gtatgcacct 20
<210> 32
   <211> 20
   <212> DNA
   <213> Artificial Sequence
<220>
   <223> Description of Artificial Sequence: artificial sequence
<400> 32
   agagccattg tctacagagg 20
<210> 33
   <211> 20
   <212> DNA
   <213> Artificial Sequence
<220>
   <223> Description of Artificial Sequence: artificial
<400> 33
   acaatacctc actaagagtg 20
<210> 34
   <211> 21
   <212> DNA
   <213> Artificial Sequence
<220>
   <223> Description of Artificial Sequence: artificial
<400> 34
   gtctcagttt ctgtctcctt c 21
<210> 35
   <211> 19
   <212> DNA
   <213> Artificial Sequence
<220>
   <223> Description of Artificial Sequence: artificial
<400> 35
   tgaacatgca ctaccacca 19
<210> 36
   <211> 20
   <212> DNA
   <213> Artificial Sequence
<220>
   <223> Description of Artificial Sequence: artificial
<400> 36
   atccttgcat gtgtatgagc 20
<210> 37
   <211> 20
   <212> DNA
   <213> Artificial Sequence
<220>
   <223> Description of Artificial Sequence: artificial
<400> 37
   tgaaccagga gtagcaagag 20
<210> 38
   <211> 23
   <212> DNA
   <213> Artificial Sequence
<220>
   <223> Description of Artificial Sequence: artificial
<400> 38
   gtgattattc attaattggg ttc 23
<210> 39
   <211> 21
   <212> DNA
   <213> Artificial Sequence
<220>
   <223> Description of Artificial Sequence: artificial
<400> 39
   tgcaatggtt gattgatgct c 21
<210> 40
   <211> 18
   <212> DNA
   <213> Artificial Sequence
<220>
   <223> Description of Artificial Sequence: artificial
<400> 40
   acattcactt gctcacct 18
<210> 41
   <211> 18
   <212> DNA
   <213> Artificial Sequence
<220>
   <223> Description of Artificial Sequence: artificial
<400> 41
   gtaaatacca cttgacca 18
<210> 42
   <211> 18
   <212> DNA
   <213> Artificial Sequence
<220>
   <223> Description of Artificial Sequence: artificial
<400> 42
   tgccgacatg tgatgtct 18
<210> 43
   <211> 18
   <212> DNA
   <213> Artificial Sequence
<220>
   <223> Description of Artificial Sequence: artificial
<400> 43
   aggtgagcaa gtgaatgt 18
<210> 44
   <211> 42
   <212> DNA
   <213> Homo sapiens
<400> 44
   agtgtcagat gacacagcac agcaagaccg aggcccttgg tt 42
<210> 45
   <211> 41
   <212> DNA
   <213> Homo sapiens
<400> 45
   agagacatat gggagtccag tagacatcaa tcaaactgga c 41
<210> 46
   <211> 41
   <212> DNA
   <213> Homo sapiens
<400> 46
   caccagttct gcatctcttg ctcctccttc tgtttctccg a 41
<210> 47
   <211> 41
   <212> DNA
   <213> Homo sapiens
<400> 47
   gtacccacca tcacgcccgg ttaatttttg tgtttttagt a 41
<210> 48
   <211> 41
   <212> DNA
   <213> Homo sapiens
<400> 48
   gtggggaatg gatgaaattt tataacaggg tgcagaggca g 41
<210> 49
   <211> 41
   <212> DNA
   <213> Homo sapiens
<400> 49
   tcttgctact cctggttcag cgccacccta acacccatga c 41
<210> 50
   <211> 41
   <212> DNA
   <213> Homo sapiens
<400> 50
   ttcagcgcca ccctaacacc catgaccgcc tcccaccagg g 41
<210> 51
   <211> 41
   <212> DNA
   <213> Homo sapiens
<400> 51
   ttcacggtac acctgggacc gaggcccgtg gtcatgctgt g 41
<210> 52
   <211> 41
   <212> DNA
   <213> Homo sapiens
<400> 52
   ggtgcatcag ggaagggagt atatgggagg aagaaggact c 41
<210> 53
   <211> 41
   <212> DNA
   <213> Homo sapiens
<400> 53
   acttcagtct gtgtccttga cctgctgctt cttcctaggg g 41
<210> 54
   <211> 41
   <212> DNA
   <213> Homo sapiens
<400> 54
   gaccccacct tcctcttcca gtccattacc gccaacatca t 41
<210> 55
   <211> 41
   <212> DNA
   <213> Homo sapiens
<400> 55
   aggcaagttt acaaaaacct gcaggaaatc aatgcttaca t 41
<210> 56
   <211> 41
   <212> DNA
   <213> Homo sapiens
<400> 56
   ctgcaggaaa tcaatgctta cattggccac agtgtggaga a 41
<210> 57
   <211> 41
   <212> DNA
   <213> Homo sapiens
<400> 57
   cgtgaaaccc tggaccccag cgcccccaag gacctcatcg a 41
<210> 58
   <211> 39
   <212> DNA
   <213> Homo sapiens
<400> 58
   cctggacccc agcgccccca aggacctcat cgacaccta 39
<210> 59
   <211> 41
   <212> DNA
   <213> Homo sapiens
<400> 59
   tacccccaac ataccagatc cgcttcctgc cccgctgaag g 41
<210> 60
   <211> 41
   <212> PRT
   <213> Homo sapiens
<400> 60
   Glu Leu Ser Val Leu Leu Phe Leu Ala Leu Leu Thr Gly Leu Leu Leu
    1               5                  10                  15
   Leu Leu Val Gln Arg His Pro Asn Thr His Asp Arg Leu Pro Pro Gly
               20                  25                  30
   Pro Arg Pro Leu Pro Leu Leu Gly Asn
           35                  40
<210> 61
   <211> 41
   <212> PRT
   <213> Homo sapiens
<400> 61
   Cys Leu Ile Glu Glu Leu Arg Lys Ser Lys Gly Ala Leu Met Asp Pro
    1               5                  10                  15
   Thr Phe Leu Phe Gln Ser Ile Thr Ala Asn Ile Ile Cys Ser Ile Val
               20                  25                  30
   Phe Gly Lys Arg Phe His Tyr Gln Asp
           35                  40
<210> 62
   <211> 41
   <212> PRT
   <213> Homo sapiens
<400> 62
   Gln Glu Ile Asn Ala Tyr Ile Gly His Ser Val Glu Lys His Arg Glu
    1               5                  10                  15
   Thr Leu Asp Pro Ser Ala Pro Lys Asp Leu Ile Asp Thr Tyr Leu Leu
               20                  25                  30
   His Met Glu Lys Glu Lys Ser Asn Ala
           35                  40
<210> 63
   <211> 41
   <212> PRT
   <213> Homo sapiens
<400> 63
   Asn Ala Tyr Ile Gly His Ser Val Glu Lys His Arg Glu Thr Leu Asp
    1               5                  10                  15
   Pro Ser Ala Pro Lys Asp Leu Ile Asp Thr Tyr Leu Leu His Met Glu
               20                  25                  30
   Lys Glu Lys Ser Asn Ala His Ser Glu
           35                  40
<210> 64
   <211> 25
   <212> PRT
   <213> Homo sapiens
<400> 64
   Asp Ile Asp Leu Thr Pro Gln Glu Cys Gly Val Gly Lys Ile Pro Pro
      1               5                  10                  15
   Thr Tyr Gln Ile Arg Phe Leu Pro Arg
               20                  25